(19) Europäisches Patentamt
European Patent Office
Office européen des brevets

(11) **EP 1 644 087 B1**

(12) **FASCICULE DE BREVET EUROPEEN**

(45) Date de publication et mention
de la délivrance du brevet:
**06.04.2011 Bulletin 2011/14**

(51) Int Cl.:
*A61Q 19/06* (2006.01)  *A61K 8/49* (2006.01)
*A61K 8/97* (2006.01)  *A61K 8/99* (2006.01)

(21) Numéro de dépôt: **04707249.1**

(22) Date de dépôt: **02.02.2004**

(86) Numéro de dépôt international:
**PCT/FR2004/000232**

(87) Numéro de publication internationale:
**WO 2004/093840 (04.11.2004 Gazette 2004/45)**

(54) **PROCEDE DE MISE EN EVIDENCE DE L' ACTIVITE DRAINANTE D'UN TRAITEMENT COSMETIQUE ET COMPOSITION A ACTIVITE DRAINANTE**

VERFAHREN ZUR ANZEIGE DER DRAINAGE-AKTIVITÄT EINER KOSMETISCHEN BEHANDLUNG UND ZUSAMMENSETZUNG MIT DRAINAGE-AKTIVITÄT

METHOD FOR DEMONSTRATING THE DRAINING ACTIVITY OF A COSMETIC TREATMENT, AND COMPOSITION HAVING A DRAINING ACTIVITY

(84) Etats contractants désignés:
**AT BE BG CH CY CZ DE DK EE ES FI FR GB GR HU IE IT LI LU MC NL PT RO SE SI SK TR**

(30) Priorité: **09.04.2003 FR 0304399**

(43) Date de publication de la demande:
**12.04.2006 Bulletin 2006/15**

(73) Titulaire: **L'Oréal**
**75008 Paris (FR)**

(72) Inventeurs:
• **QUERLEUX, Bernard**
**F-94170 Perreux (FR)**
• **BAZIN, Roland**
**F-91570 Bievres (FR)**

(74) Mandataire: **Tanty, François**
**Nony & Associés**
**Conseils en Propriété Industrielle**
**3, rue de Penthièvre**
**75008 Paris (FR)**

(56) Documents cités:
**EP-A- 1 038 518     EP-A- 1 181 926**

• **S. RICHARD ET AL.: "Characterization of the skin in vivo by high resolution magnetic resonance imaging: water behaviour and age-related effects" JOURNAL OF INVESTIGATIVE DERMATOLOGY, vol. 100, mai 1993 (1993-05), pages 705-709, XP002288520 cité dans la demande**
• **F. FRANSCONI, S. AKOKA, J. GUESNET, J.M. BARET, D. DERSIGNY, B. BREDA, C. MULLER, P. BEAU: "Measurement of epidermal moisture content by magnetic resonance imaging: assessment of a hydratation cream" BRITISH JOURNAL OF DERMATOLOGY, vol. 132, 1995, pages 913-917, XP009033694 -& B. QUERLEUX, J. BITTOUN, C. MULLER, S. AKOKA, F. FRANCONI, J. GUESNET, J.M. BARRET, D. DERSIGNY, B. BREDA, P. BEAU: "Correspondence - Measurement of epidermial moisture content. And reply" BRITISH JOURNAL OF DERMATOLOGY, vol. 135, 1996, pages 144-145, XP002288521**
• **B. QUELREUX, C. CORNILLON, O. JOLIVET, J. BITTOUN: "Anatomy annd physiology of subcutaneous adipose tissue by in vivo magnetic resonance imaging and spectroscopy: Relationships with sex and presence of cellulite" SKIN RESEARCH AND TECHNOLOGY, vol. 8, 2002, pages 118-124, XP002288522**

## Description

La présente invention concerne le domaine de la cosmétique et/ou de la dermo-cosmétique et notamment celui des compositions ayant une action drainante, telles que certains produits amincissants ou destinés à faciliter la disparition de la peau d'orange. L'invention s'intéresse à l'étude des effets de ces compositions cosmétiques et/ou dermo-cosmétiques. Par " composition cosmétique " on entend au sens de la présente une composition telle que définie dans la Directive 93/35/CEE du 14 juin 1993, modifiant la Directive 76/768/CEE. Dans la suite, on utilisera le terme "composition cosmétique" pour désigner à la fois des compositions cosmétiques et/ou dermo-cosmétiques.

De nombreuses compositions cosmétiques revendiquant une action amincissante sont connues, celles-ci agissant en limitant la lipogénèse ou en favorisant la lipolyse.

Il peut s'avérer assez difficile de mettre en évidence les effets induits par l'application d'une composition revendiquant une action drainante, notamment amincissante, et notamment de les quantifier.

Il peut s'avérer également difficile de mettre en évidence des effets quantifiables sur une période de temps relativement courte.

L'invention vise à proposer un procédé permettant de mettre en évidence l'activité drainante d'un traitement cosmétique comportant l'utilisation d'une composition cosmétique.

Au sens de la présente invention, par " activité drainante " on désigne une action de réduction de la rétention d'eau dans l'épiderme et/ou le derme superficiel.

L'invention a ainsi pour objet, selon un premier de ses aspects, un procédé pour mettre en évidence l'activité drainante d'un traitement cosmétique comportant l'application topique d'une composition amincissante ou destinée à faciliter la disparition de la peau d'orange et comportant un actif lypolytique, ce procédé comportant :

- l'acquisition avant le traitement d'au moins une première donnée représentative de la teneur en eau dans le derme superficiel et/ou dans l'épiderme, par une technique d'imagerie IRM à haute résolution spatiale,
- le traitement d'au moins une partie du corps avec la composition, par voie topique
- l'acquisition après le traitement d'au moins une deuxième donnée représentative de la teneur en eau dans le derme superficiel et/ou l'épiderme, par ladite technique d'imagerie IRM,
- la mise en évidence d'une activité drainante éventuelle par comparaison des première et deuxième données.

Par " technique d'imagerie IRM à haute résolution spatiale " on désigne une technique d'imagerie IRM, notamment du proton, présentant une résolution spatiale suffisante pour distinguer sur l'image IRM obtenue l'épiderme du derme superficiel. Typiquement, une résolution spatiale en profondeur suffisante est de l'ordre de 50 $\mu$m ou mieux, et de préférence, 35 $\mu$m ou mieux.

Par " derme superficiel ", on désigne la partie du derme qui s'étend entre l'épiderme et le derme profond où peuvent s'étendre des indentations du tissu adipeux. Le derme superficiel peut se situer par exemple, sur certains individus, à une profondeur comprise entre 50 $\mu$m et 500 $\mu$m par rapport à la surface de la peau.

Le traitement ci-dessus comporte l'application topique de la composition, cette dernière pouvant comporter au moins un actif ayant une action sur la rétention d'eau dans les couches superficielles de la peau. Par " couches superficielles de la peau " on désigne le derme superficiel et l'épiderme.

Le traitement peut avantageusement comporter en outre, le cas échéant, un massage, notamment un massage permettant d'agir sur la circulation lymphatique.

Pour mettre en évidence l'activité drainante, on peut avantageusement comparer N(H) avant et après le traitement. N(H) désigne la densité relative de protons et correspond à la fraction de protons détectable par imagerie par résonance magnétique.

Le paramètre N(H) est bien connu de l'homme de l'art de l'IRM et il est défini notamment dans l'article "Characterization of the skin in vivo by high resolution magnetic resonance imaging: water behaviour and age-related effects ", S. Richard et al, The Journal of Investigative Dermatology, vol.100 No.5 May 1993,

D'autres paramètres toutefois sont représentatifs de la teneur en eau. Ainsi, en est-il notamment des paramètres T1 et T2 qui sont descriptifs des interactions entre les protons, en particulier ceux de l'eau, et leur environnement Tout particulièrement une augmentation de teneur en eau est souvent associée à une augmentation du T1, comme il est rapporté par exemple dans l'article : "In vivo brain water determination by T1 measurements : effect of total water content, hydration fraction, and field strength ", PP Fatouros et al, Magnetic Resonance in Medicine 17: 402-413, 1991.

Ces paramètres T1 et/ou T2 peuvent être utilisés indépendamment du N(H) ou en complément du N(H).

Le traitement comporte l'application topique cutanée d'une composition comportant un actif lipolytique, notamment l'un de ceux identifiés ci-après.

Le procédé ci-dessus peut permettre de mettre en évidence une activité drainante inattendue liée à l'utilisation de certains actifs, dont l'action sur la teneur en eau des couches superficielles de la peau ne peut être anticipée *a priori.*

La composition contient dans un milieu cosmétiquement acceptable au moins un actif lipolytique, ledit actif étant tel, que lorsqu'il est présent en quantité suffisante dans la composition, cette dernière ermette notamment d'entraîner une diminution de N(H) d'au moins 2,5 % dans le derme superficiel et/ou l'épiderme et/ou d'au moins 2% pour le T1, et/ou d'au moins 1,5% pour le T2 dans l'épiderme. La diminution de N(H) peut notamment être d'au moins 4 % dans le derme superficiel et/ou l'épiderme, mieux d'au moins 9 % dans le derme superficiel. La concentration optimale pourra être déterminée expérimentalement, notamment par des mesures successives de T1 et/ou T2 et/ou N(H).

L'activité drainante pourra être due essentiellement à un seul actif ou en variante à plusieurs actifs, dont la concentration individuelle pourra être plus faible.

Par " cosmétiquement acceptable " on désigne une composition compatible au moins avec la peau.

Avantageusement, le pouvoir drainant de la composition est suffisamment fort pour que la diminution de N(H), et/ou T1, et/ou T2 définie ci-dessus puisse être observée sur une durée de traitement relativement courte, par exemple égale à quatre semaines, avec par exemple des applications quotidiennes matin ou soir.

La composition peut comporter au moins un actif choisi parmi la caféine et ses dérivés, le citrate de caféine, la théophylline et ses dérivés, la théobromine, l'acéfylline, l'aminophylline, la chloroéthylthéophylline, le diprofylline, le diniprophylline, l'étamiphylline et ses dérivés, l'étofylline, la proxyphylline, l'éphédrine et ses dérivés, les associations de caféine et de silanol, les composés d'origine naturelle contenant des bases xanthiques tels que les extraits de thé, de café, de guarana, de maté, de cola (*Cola Nitida*) *;* les extraits végétaux de *Garcinia Cambogia,* les extraits de *Bupleurum chinensis,* les extraits de lierre grimpant (*Hedera Helix*), d'arnica (*Arnica Montana L*)*,* de romarin (*Rosmarinus officinalis L*)*,* de souci (*Calendula officinales*)*,* de sauge (*Salvia officinalis L*)*,* de ginseng (*Panax ginseng*)*,* de millepertuis *(Hypericum Perforatum),* de fragon *(Ruscus aculeatus L)* d'ulmaire *(Filipendula ulmaria L),* d'orthosiphon *(Orthosiphon Stamincus Benth),* de bouleau *(Betula alba),* de cécropia et d'arganier, les extraits de ginkgo biloba, les extraits de prêles, les extraits d'escine, les complexes de phospholipide et de proanthocyanidines d'écorce de marron d'inde, les extraits de cangzhu, les extraits de *chrysanthemum indicium,* les sapogénines telles que la diosgénine ou l'hécogénine, leurs dérivés et les extraits naturels en contenant, en particulier le Wild Yam, les extraits des plantes du genre *Armeniacea, Atractylodis Platicodon, Sinom-menum, Pharbitidis, Flemingia,* les extraits de *Coleus* tels que *C. Forskohlii, C. blumei, C. esquirolii, C. scutellaroïdes, C. xanthantus* et *C. Barbatus,* les extraits de Ballote, les extraits de *Guioa,* de *Davallia,* de *Terminalia,* de *Barringtonia,* de *Trema, d'Antirobia,* les extraits d'algues ou de phytoplancton tels que le rhodystérol ou l'extrait de *Laminaria Digitata,* l'algue skeletonema et les diatomées.

La composition peut notamment comporter au moins un extrait de Dioscorée riche en diosgénine, provenant par exemple de racines d'igname sauvage. Cet extrait ou tout autre actif ayant une activité drainante peut être par exemple présent dans la composition conjointement à au moins un glycéride d'acide gras ou de mélange d'acides gras en $C_6$ à $C_{22}$, éventuellement polyoxyéthyléné et/ou polyoxypropyléné.

On pourra par exemple choisir un extrait de racines de *Dioscorea opposita* en solution dans un mélange d'un dérivé de polyéthylèneglycol (6OE) et de mono-, di-, et triglycéride d'acides caprylique et caprique / conservateurs / glycérine (rapport pondéral 1/93,8/0,2/5), commercialisé sous la dénomination " Dioschol " par la société SEDERMA, notamment dans une concentration supérieure ou égale à 5 %, mieux 8 %, par rapport au poids total de la composition.

L'invention pourra être mieux comprise à la lecture de la description détaillée qui va suivre, et à l'examen du dessin annexé, sur lequel :

- la figure 1 illustre de manière schématique l'acquisition d'une image IRM sur un individu,
- la figure 2 représente un exemple d'image IRM,
- la figure 3 représente un exemple d'évolution du signal IRM dans une région d'intérêt au sein d'une couche cutanée en fonction du temps de répétition Tr de la séquence, permettant le calcul par approximation exponentielle du T1,
- la figure 4 représente un exemple d'évolution du signal IRM dans une région d'intérêt au sein d'une couche cutanée en fonction du temps d'écho Te de la séquence, permettant le calcul par approximation exponentielle du T2 , et
- les figures 5 à 7 illustrent un exemple de technique d'application de la composition.

Pour mettre en évidence l'activité drainante d'une composition cosmétique appliquée sur la peau, on peut procéder à l'acquisition d'images IRM selon le protocole de mesure suivant.

## Protocole de mesure

L'appareil d'imagerie par résonance magnétique du proton utilisé est par exemple celui de référence SIGNA 1,5 Tesla, commercialisé par la société General Electric.

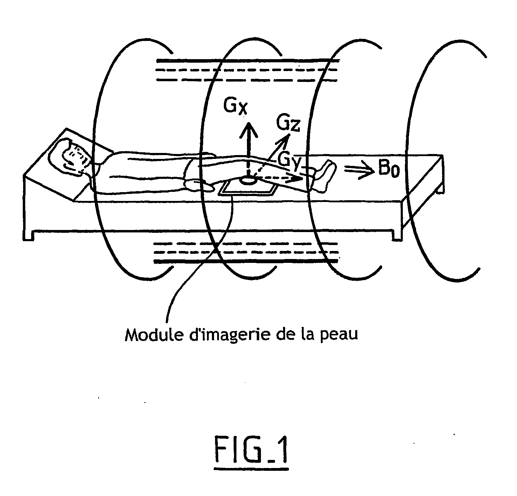

L'individu objet du test est allongé dans l'appareil comme illustré à la figure 1. Sur cette dernière $G_x$, $G_y$ et $G_z$ désignent, de manière conventionnelle, l'intensité de gradient dans les trois directions.

L'appareil peut, s'il ne dispose pas dès l'origine de la résolution spatiale suffisante dans le sens de la profondeur, par exemple 35 $\mu$m ou mieux, être équipé d'un module d'imagerie de la peau tel que décrit dans la demande de brevet français FR 2 612 641, ce module étant destiné à améliorer la résolution spatiale des images IRM. Un exemple d'utilisation d'un tel module est décrit dans l'article "In vivo proton relaxation times analysis of the skin layers by Magnetic Resonance Imaging", S. Richard et al, The Journal of Investigative Dermatology, vol. 97, No. 1 July 1991, 120-125.

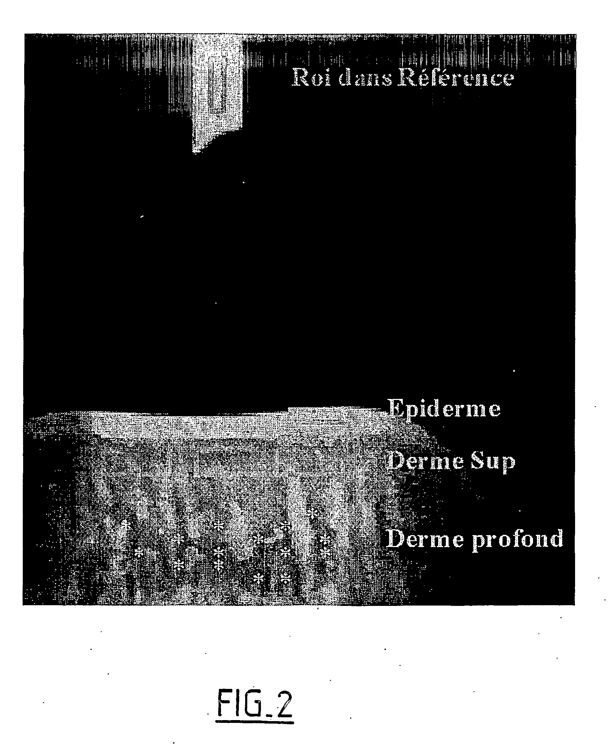

Un petit tube en matériau non magnétique rempli d'eau déminéralisée, est placé à proximité de la région étudiée, de façon à apparaître sur l'image IRM et servir de référence, comme on peut le voir sur la figure 2.

Pour chaque image, différentes mesures peuvent être effectuées dans des régions d'intérêt différentes. Par " région d'intérêt ou par abréviation Roi " on désigne une zone de l'image dans laquelle on effectue une mesure de l'intensité moyenne du signal.

Référence

La région d'intérêt est délimitée par un seul rectangle, comme on peut le voir sur la figure 2.

Epiderme

La région d'intérêt est délimitée par trois rectangles disposés sensiblement bout à bout, comme illustré sur la figure 2, dans l'épaisseur de l'épiderme.

Derme superficiel

La région d'intérêt est délimitée par trois rectangles disposés sensiblement bout à bout entre l'épiderme et le derme profond, où s'étendent les indentations du tissu adipeux.

Calcul des temps de relaxation T1 et T2

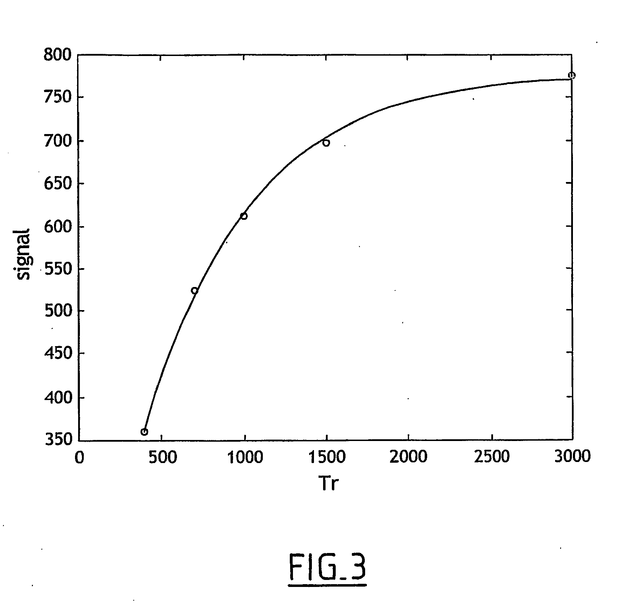

Pour chaque région d'intérêt, cinq images sont acquises en faisant varier le temps de répétition $T_r$, avec $T_r$ respectivement égal à 3000, 1500, 1000, 700 et 400 ms. Un exemple de courbe obtenue a été représenté à la figure 3. La valeur du temps de relaxation T1 qui décrit le mieux la variation exponentielle observée est calculée, de manière connue en soi.

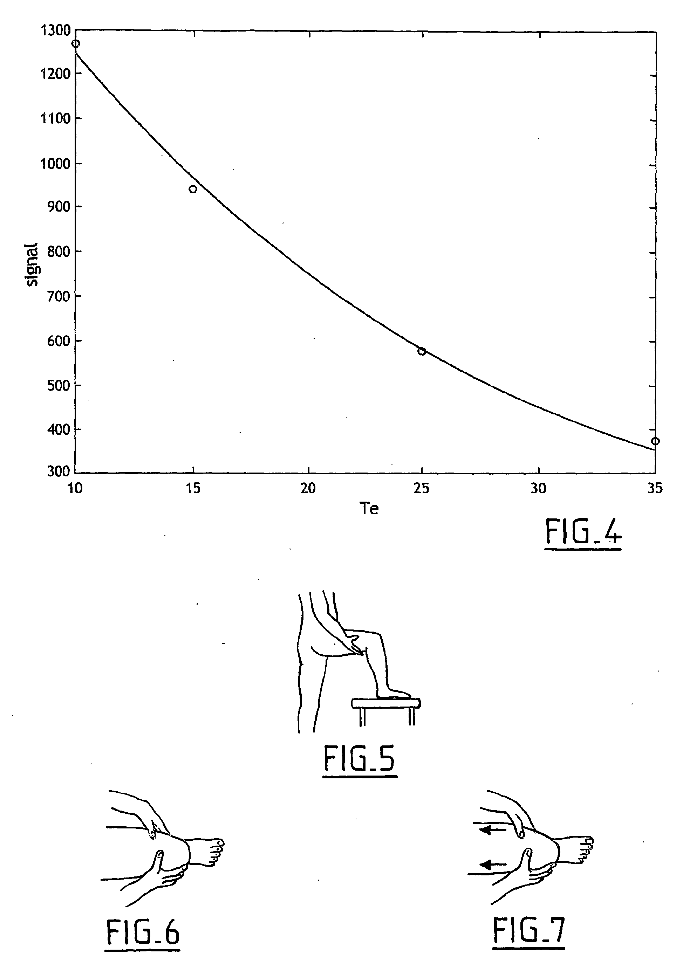

Quatre images sont également acquises pour mesurer le temps de relaxation T2, en faisant varier le temps d'écho $T_e$, celui-ci étant par exemple égal successivement à 10, 15, 25 et 35 ms. Un exemple de courbe obtenue a été représenté à la figure 4. La valeur du temps de relaxation T2 qui décrit le mieux la variation exponentielle observée est calculée, de manière connue en soi.

Puis on calcule pour chaque région d'intérêt (épiderme ou derme superficiel) la densité de protons *Rho* et relative N(H) à partir des formules suivantes :

$$Rho = \frac{S_{(Te=10\,ms,\,Tr \approx 3000\,ms)}}{\exp(-\frac{10}{T_2}) * (1 - \exp(-\frac{3000}{T_1}))}$$

$$N(H) = \frac{Rho_{\text{Région d'intérêt}}}{Rho_{\text{Référence}}}$$

Dans la formule ci-dessus, S désigne l'intensité moyenne du signal dans la région d'intérêt en question, pour l'image acquise avec $T_e$ = 10 ms et $T_r$ = 3000 ms.

La densité de protons *Rho* peut être considérée comme représentative de la teneur en eau mais dépend encore d'un facteur lié aux conditions d'acquisition, alors que N(H) ne dépend plus que de la teneur en eau dans le tissu étudié. La normalisation permet une comparaison entre les individus ou pour chaque individu à des temps différents.

Ainsi, une diminution de N(H) peut permettre de mettre en évidence une diminution de la teneur en eau dans les couches superficielles de la peau, donc un effet drainant.

De même, une diminution du T1 ou du T2 peut permettre de mettre en évidence une diminution de la teneur en eau dans les couches superficielles de la peau, donc un effet drainant.

Essais

On a mesuré T1 et T2 et calculé N(H) selon le protocole de mesure ci-dessus dans le derme superficiel et l'épiderme sur 20 volontaires féminins âgés de 19 à 45 ans, présentant une surcharge graisseuse localisée inesthétique, telle que de la cellulite au niveau des cuisses, visible à l'oeil nu, ainsi qu'un indice de QUETELET compris entre 20 et 27. L'indice de QUETELET est le rapport $P/T^2$, où P le poids en kg et T la taille exprimée en m.

La composition dont on cherche à évaluer les effets est appliquée de manière quotidienne, matin ou soir, pendant un mois, sur les hanches et sur les jambes, du haut de la cuisse jusqu'aux genoux, selon une gestuelle d'application prédéfinie, durant à peu près trois minutes, illustrée aux figures 5 à 7.

L'individu commence par surélever un pied en le posant sur un objet tel que par exemple une chaise ou une baignoire, de manière à prendre une position confortable, comme illustré à la figure 5.

Ensuite, l'individu plaque ses mains de chaque côté de son genou et pose ses pouces sur l'avant de sa cuisse, comme illustré à la figure 6.

L'individu peut alors remonter ses mains et lisser sa cuisse avec un mouvement rapide et appuyé, l'une des mains étant déplacée jusqu'en haut de la fesse, comme illustré à la figure 7.

Enfin, les mains sont déplacées alternativement sur l'avant et l'arrière de la cuisse jusqu'à pénétration complète de la composition.

Une acquisition des paramètres T1, T2 et N(H) pour l'épiderme et le derme superficiel est effectuée initialement, avant toute application, puis après quatre semaines de suivi du traitement.

La composition appliquée lors des essais présente une formulation (% en poids par rapport au poids total de la composition) similaire à celle donnée ci-dessous.

| Phase aqueuse | |
|---|---|
| Eau | Qs 100 |
| Caféine | 3 |
| Extrait végétaux | 0,2 |
| Acide salicylique | 0,72 |
| Sulfate de Mg | 0,7 |
| Citrate trisodique | 2 |
| Glycérine | 8 |
| Butylène glycol | 5 |
| Dioschol(1) | 3 |
| Eau thermale | 5 |
| Ethanol | 20 |
| Conservateurs | 0,5 |
| Colorants | 0,0001 |
| Neutralisant | 0,72 |
| | |
| **Phase huileuse** | |
| Cyclopentasiloxane | 9 |
| Isoparaffine | 2 |
| Cyclohexasiloxane | 5 |
| Parfum | 0,3 |

(suite)

| Phase huileuse | |
|---|---|
| DC2-5225C(2) | 8 |
| (1) Dioschol : Extrait de racines de *Dioscorea opposita* (igname sauvage) dans un mélange d'un dérivé de polyéthylèneglycol (60E) et de mono-, di-, et triglycéride d'acides caprylique et caprique / conservateurs / glycérine (rapport pondéral 1/93,8/0,2/5) commercialisé par la société SEDERMA. (2) : DC2-5225C : Mélange de polydiméthylsiloxane oxyéthyléné oxypropyléné (18OE/18OP), de cyclopentasiloxane et d'eau (rapport pondéral 10/88/2) commercialisé par la société DOW CORNING. | |

Les phases aqueuse et huileuse sont préparées séparément à froid, puis la phase aqueuse est ensuite dispersée sous agitation vigoureuse dans la phase huileuse.

La significativité des résultats est déterminée pour T1 et N(H) par un test dé Student appareillé. Pour T2, la significativité des résultats est déterminée par analyse de la covariance à effets mixtes avec le facteur temps comme facteur fixe (facteur expérimental), la mesure du T2 dans la référence au même temps comme covariable et le facteur témoin comme facteur aléatoire.

On a constaté lors des essais qu'il n'y avait pas de variation significative des valeurs T1, T2 et N(H) de la référence entre le début et la fin du traitement.

Epiderme

| | Début | Fin | Evolution Début/Fin | |
|---|---|---|---|---|
| T1 (ms) | 735 +/- 82 | 711 +/- 85 | p=0,05 | ~ -3,3 % |
| T2 (ms) | 22,1+/-1,2 | 21.8+/-1,1 | p=0,004 | ~ -1,5% |
| N(H) (u.a)* | 0,66 +/- 0,03 | 0,63 +/- 0,03 | p=0,003 | ~ -4,5% |
| *u.a. : unité arbitraire | | | | |

Derme superficiel

| | Début | Fin | Evolution Début/Fin | |
|---|---|---|---|---|
| T1 (ms) | 691 +/- 44 | 677 +/- 46 | p=0,03 | ~ -2,0% |
| T2 (ms) | 12,8 +/- 0,8 | 12,7 +/- 0,8 | Non significatif | ~ -0,8% |
| N(H) | 0,42 +/- 0,05 | 0,38 +/- 0,04 | P<0,0001 | ~ -9,5% |
| p désigne le risque d'erreur et il est souvent fixé à 5% soit p<0,05 | | | | |

On constate une diminution statistiquement significative de la teneur en eau dans les couches superficielles de la peau. Cette diminution est représentative d'une activité drainante.

Le protocole de mesure ci-dessus permet de quantifier l'activité drainante d'une composition en terme de variation notamment de N(H), de T1 ou de T2. On peut ainsi mettre en évidence une activité inattendue sur les couches superficielles de la peau d'une composition cosmétique comportant un actif lipolytique.

L'invention n'est pas limitée à une composition comportant un actif particulier et concerne toute composition cosmétique comportant au moins un actif lipolytique, ayant une activité drainante pouvant se traduire par une diminution relativement élevée de N(H) et/ou T1 et/ou T2.

La composition pourra comporter au moins un actif ayant soit une action sur la phosphodiestérase, en l'inhibant, sur des récepteurs à inhiber tels que les β-2 bloqueurs, les NPY-bloqueurs (notamment ceux décrits dans le brevet EP 838 217), soit sur la synthèse des récepteurs aux LDL ou VLDL, soit stimulant les récepteurs β et les protéines G, conduisant à l'activation de l'adénylcyclase.

La composition pourra encore comporter un peptide, notamment un peptide dérivé de l'hormone parathyroïdienne tel que décrit dans FR 2 788 058, FR 2 781 231, ou un peptide décrit dans FR 2 786 693, voire tout autre peptide ayant des propriétés lipolytiques.

La composition pourra encore comporter une protamine et ses dérivés, par exemple une protamine telle que décrite dans FR 2 758 724.

Bien entendu, la composition pourra comporter, outre au moins un actif revendiquant un effet drainant, présent dans une quantité allant par exemple de 0,001 à 20 % et de préférence de 0,1 à 10 % en poids par rapport au poids total de la composition, d'autres composés et notamment les adjuvants habituels dans le domaine cosmétique et/ou dermatologique, tels que les conservateurs, les antioxydants, les agents complexants, les solvants, les parfums, les charges, les filtres UV, les bactéricides, les absorbeurs d'odeur, les matières colorantes et les vésicules lipidiques, cette liste n'étant pas limitative.

La composition pourra être conditionnée dans un conditionnement en matière thermoplastique ou non, tel que par exemple un pot, un flacon ou un tube, dans une quantité comprise par exemple entre 5 ml et 250 ml.

Le cas échéant; la composition pourra être conditionnée dans un dispositif permettant d'exercer une action de massage de la peau lors de l'application.

La composition pourra être conditionnée avec des instructions concernant un massage à effectuer au moment de l'application, ces instructions figurant par exemple sur l'emballage lui-même ou sur un élément distinct, par exemple une notice ou un support informatique.

Dans toute la description, y compris les revendications, l'expression " comportant un " doit être comprise comme étant synonyme comme " comportant au moins un ", sauf si le contraire est spécifié.

**Revendications**

1. Procédé pour mettre en évidence l'activité drainante d'un traitement cosmétique comportant l'application topique d'une composition amincissante ou destinée à faciliter la disparition de la peau d'orange et comportant un actif lypolytique, ce procédé comportant :

   - l'acquisition avant le traitement d'au moins une première donnée représentative de la teneur en eau dans le derme superficiel et/ou dans l'épiderme, par une technique d'imagerie IRM à haute résolution spatiale,
   - le traitement d'au moins une partie du corps avec la composition,
   - l'acquisition après traitement d'au moins une deuxième donnée représentative de la teneur en eau dans le derme superficiel et/ou dans l'épiderme, par ladite technique d'imagerie IRM,
   - la mise en évidence d'une activité drainante éventuelle par comparaison des première et deuxième données.

2. Procédé selon l'une des revendications précédentes, **caractérisé par le fait que** l'on compare N(H) avant et après le traitement.

3. Procédé selon l'une des revendications 1 à 3, **caractérisé par le fait que** l'on compare le temps de relaxation T1 avant et après le traitement.

4. Procédé selon l'une des revendications 1 à 3, **caractérisé en ce que** l'on compare le temps de relaxation T2 avant et après le traitement.

**Claims**

1. A method of demonstrating the draining activity of a cosmetic treatment, which method comprises a lipolytic active ingredient and the topical application of a slimming composition or of a composition intended for facilitating disappearance of orange peel skin, said method comprising:

   · prior to the treatment, acquiring at least one first datum representative of the water content in the superficial dermis and/or in the epidermis, using an MRI imaging technique having high spatial resolution;
   · treating at least part of the body with the composition;
   · after the treatment, acquiring at least one second datum representative of the water content in the superficial dermis and/or in the epidermis, using said MRI imaging technique;
   · demonstrating any draining activity by comparing the first and second data.

2. A method according to the preceding claim, **characterized by** the fact that N(H) is compared before and after treatment.

3. A method according to claim 1 or claim 2, **characterized by** the fact that the relaxation time T1 is compared before and after the treatment.

4. A method according to any one of claims 1 to 3, **characterized in that** the relaxation time T2 is compared before and after the treatment.


**Patentansprüche**

1. Verfahren zur Anzeige der Drainage-Aktivität einer kosmetischen Behandlung umfassend das topische Aufbringen einer Zusammensetzung, die schlank macht oder die dazu bestimmt ist, das Verschwinden der Orangenhaut zu unterstützen und die einen lipolytischen Wirkstoff umfasst, wobei dieses Verfahren folgendes umfasst:

   - Sammeln von mindestens ersten Daten vor der Behandlung, die für den Gehalt an Wasser in der Oberflächenhaut und/oder der Epidermis repräsentativ sind, durch eine bildgebende IRM-Technik mit hoher Raumauflösung,
   - Behandlung von mindestens einem Teil des Körpers mit der Zusammensetzung;
   - Sammeln von mindestens zweiten Daten nach der Behandlung, die für den Gehalt an Wasser in der Oberflächenhaut und/oder der in der Epidermis repräsentativ sind, durch die bildgebende IRM-Technik;
   - Anzeigen einer Drainage-Aktivität gegebenenfalls durch Vergleich der ersten und zweiten Daten.

2. Verfahren nach einem der vorhergehenden Ansprüche, **dadurch gekennzeichnet, dass** N(H) vor und nach der Behandlung verglichen wird.

3. Verfahren nach einem der Ansprüche 1 bis 3, **dadurch gekennzeichnet, dass** die Relaxationszeit T1 vor und nach der Behandlung verglichen wird.

4. Verfahren nach einem der Ansprüche 1 bis 3, **dadurch gekennzeichnet, dass** die Relaxationszeit T2 vor und nach der Behandlung verglichen wird.

Module d'imagerie de la peau

# FIG.1

FIG.2

## FIG.3

FIG.4

FIG.5

FIG.6

FIG.7

## RÉFÉRENCES CITÉES DANS LA DESCRIPTION

**Documents brevets cités dans la description**

- FR 2612641
- EP 838217 A
- FR 2788058
- FR 2781231
- FR 2786693
- FR 2758724

**Littérature non-brevet citée dans la description**

- **S. Richard et al.** Characterization of the skin in vivo by high resolution magnetic resonance imaging: water behaviour and age-related effects. *The Journal of Investigative Dermatology,* Mai 1993, vol. 100 (5
- **PP Fatouros et al.** In vivo brain water determination by T1 measurements : effect of total water content, hydration fraction, and field strength. *Magnetic Resonance in Medicine,* 1991, vol. 17, 402-413
- **S. Richard et al.** In vivo proton relaxation times analysis of the skin layers by Magnetic Resonance Imaging. *The Journal of Investigative Dermatology,* Juillet 1991, vol. 97 (1), 120-125